# EUROPEAN PATENT SPECIFICATION

(11) **EP 2 621 555 B1**
(45) Date of publication and mention of the grant of the patent: **23.10.2019**
(21) Application number: 11829903.1
(22) Date of filing: 29.09.2011
(51) Int. Cl.: A61M 5/142, A61M 5/172, A61M 39/08, F04B 43/12

(54) **PRESSURE MONITORING SYSTEM FOR INFUSION PUMPS**
DRUCKÜBERWACHUNGSSYSTEM FÜR INFUSIONSPUMPEN
SYSTÈME DE SURVEILLANCE DE PRESSION POUR POMPES À PERFUSION

(30) Priority: 01.10.2010 US 388977 P
(43) Date of publication of application: 07.08.2013
(73) Proprietor: Zevex, Inc., Salt Lake City, UT 84123 (US)
(72) Inventor: BECK, Kent, Salt Lake City UT 84103 (US); EGGERS, Philip, Cottonwood Heights UT 84121 (US); WALKER, Larry, Salt Lake City UT 84123 (US)
(74) Representative: Hey, Andrew Stuart
(86) International application number: PCT/US2011/053970
(87) International publication number: WO 2012/044812

(56) References cited:
- EP-A1- 1 535 637
- US-A- 4 764 166
- US-A- 5 514 102
- US-A- 5 928 177
- US-A1- 2004 097 885
- US-B1- 6 250 164
- US-B1- 6 595 950

## Description

### THE FIELD OF THE INVENTION

The present invention relates to pressure monitoring systems in pumps. More specifically, the present invention relates to a pressure monitoring system for medical pumps such as feeding pumps and infusion pumps which allows for more accurate pressure measurement in a fluid delivery tube while utilizing inexpensive components. The pressure monitoring system isolates the pressure measurement from environmental effects such as movement of the pump or, more importantly, external forces applied to the pump such as a user grasping the pump.

### BACKGROUND

Medical pumps such as peristaltic pumps are commonly used to deliver fluids. In medical applications, peristaltic pumps and fluid delivery systems are used to deliver medication, nutrition, and other fluids to a patient. In these applications, it is important to monitor the pressure inside of the delivery tubing. Typically, pressure is measured and monitored before and after the pumping motor. This allows the pump to determine if a blockage is present in the tubing or if the pressure in the tubing is outside of a safe working range.
Measuring the pressure may also enable the pump to more accurately determine the rate of fluid delivery.

It has been difficult to accurately measure the pressure in the delivery tubing. For medical applications, a disposable tubing set is loaded into the pump and used for a relatively short period of time. This requires that the pressure monitoring system does not interfere with the loading and unloading of the tubing. Existing pressure monitoring systems have experienced inaccuracies due to the inconsistent loading or placement of the tubing or due to external forces which are applied to the pump such as when a user grabs or moves the pump.

There is a need for a pressure monitoring system for fluid delivery pumps which more accurately measures the fluid pressure inside of the tubing. There is a need for such a system which overcomes inconsistencies in tubing placement, and which is not affected by environmental conditions such as movement or forces applied to the pump.

US 6250164 discloses a method of sensing fluid pressure in which a deformable tube provides an accessible and substantially flat section. Deflection of the flat section reflects line pressure.

US 2004/097885 discloses a feeding set adaptor which is used to connect various parts of an infusion set and to integrate them with the pump to enable the monitoring of fluid pressures, the detection of bubbles, and the selective occlusion of fluid flow to prevent freeflow conditions.

### SUMMARY OF THE INVENTION

It is an object of the present invention to provide an improved pressure monitoring system. The present invention provides a pressure monitoring system as defined in the appended independent claim.

According to one aspect of the disclosure, a pressure monitoring system is provided which allows the infusion tubing to be easily loaded and unloaded from the pump. The tubing is simply placed in a channel in the pump and the door is closed. No additional latch mechanisms are necessary.

According to another aspect of the disclosure, a pressure monitoring system is provided where the pressure readings are isolated from external forces acting on the pump, and acting on the pump door in particular. The pressure monitoring system thus provides a more consistent and reliable measurement of the pressure within the tubing.

These and other aspects of the present invention are realized in a pressure monitoring system as shown and described in the following figures and related description.

### BRIEF DESCRIPTION OF THE DRAWINGS

Various embodiments of the present invention are shown and described in reference to the numbered drawings wherein:
FIG. 1 shows a perspective view of a fluid delivery pump according to the present invention;
FIG. 2 shows a perspective view of the pump of FIG. 1;
FIG. 3 shows a partial cross-sectional view of the pump of FIG. 1;
FIG. 4 shows a partial cross-sectional view of the pump of FIG. 1; and
FIGs. 5 through 7 show partial cross-sectional views of the pressure monitoring channel of pump of FIG. 1.

It will be appreciated that the drawings are illustrative and not limiting of the scope of the invention which is defined by the appended claims. The embodiments shown accomplish various aspects and objects of the invention. It is appreciated that it is not possible to clearly show each element and aspect of the invention in a single figure, and as such, multiple figures are presented to separately illustrate the various details of the invention in greater clarity. Similarly, not every embodiment need accomplish all advantages of the present invention.

### DETAILED DESCRIPTION

The invention and accompanying drawings will now be discussed in reference to the numerals provided therein so as to enable one skilled in the art to practice the present invention. The drawings and descriptions are exemplary of various aspects of the invention and are not intended to narrow the scope of the appended claims.

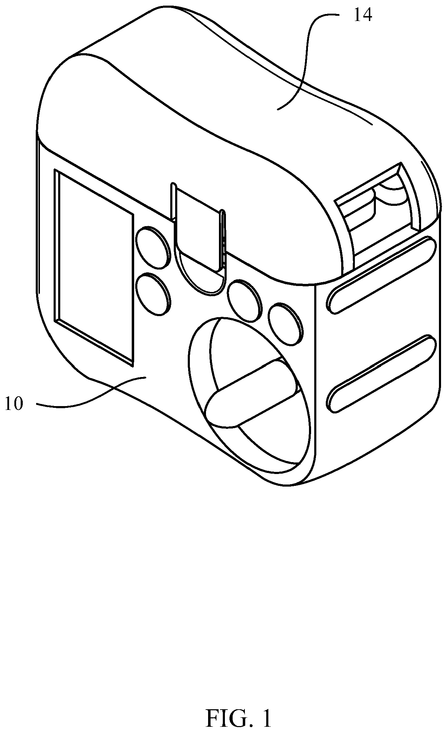

Turning now to FIG. 1, a perspective view of a pump 10 is shown. The present application applies to many types of pumps such as nutrition delivery and feeding pumps and I.V. or medication delivery pumps. For simplicity, the application simply refers to pumps or infusion pumps to indicate these types of pumps. The pump 10 is typically used for delivery of medical fluids, such as delivering medicine or nutritional solutions. Many of the controls or features of the pump 10 are known in medical peristaltic pumps, and are not discussed herein for clarity in discussing the invention. The pump 10 includes a door 14 which is closed after mounting an infusion cassette into the pump. The door 14 is used to ensure proper loading of the infusion cassette.

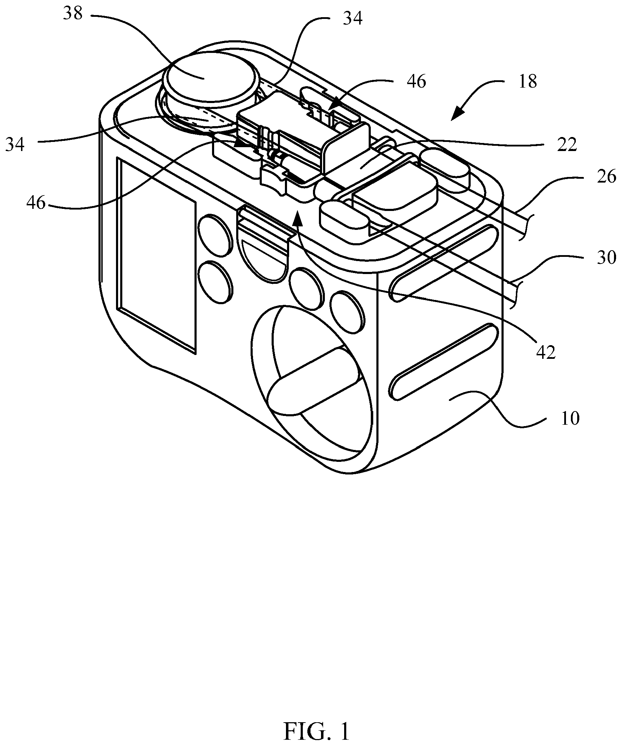

FIG. 2 shows a perspective view of the pump 10 with the door 14 removed. An infusion cassette 18 is mounted in the pump. The infusion cassette 18 includes a cassette body 22, an inflow tubing 26, an outflow tubing 30 and a pump tubing 34. The pump tubing 34 is typically flexible silicone tubing. The cassette body 22 provides connectors to attach the inflow tubing 26 to the first end of the pump tubing 34 and the outflow tubing to the second end of the pump tubing. The pump tubing thus forms a loop which is stretched around the pump rotor 38. It will be appreciated, however, that the pressure monitoring system of the present invention may also be used in other pumps such as linear peristaltic pumps.

The cassette 18 is typically loaded into the pump 10 by placing the loop of pump tubing 34 over the pump rotor 38, stretching the pump tubing, and placing the cassette body 22 into a nesting area 42. The pump includes pressure monitoring channels 46. The pressure monitoring channels 46 receive the pump tubing 34 to monitor the pressure therein. It is typically desired to monitor the pressure inside the tubing both upstream and downstream from the pump rotor 38. This allows the pump 10 to more accurately determine the fluid delivery rate and allows the pump to determine if a blockage or overpressure situation has occurred.

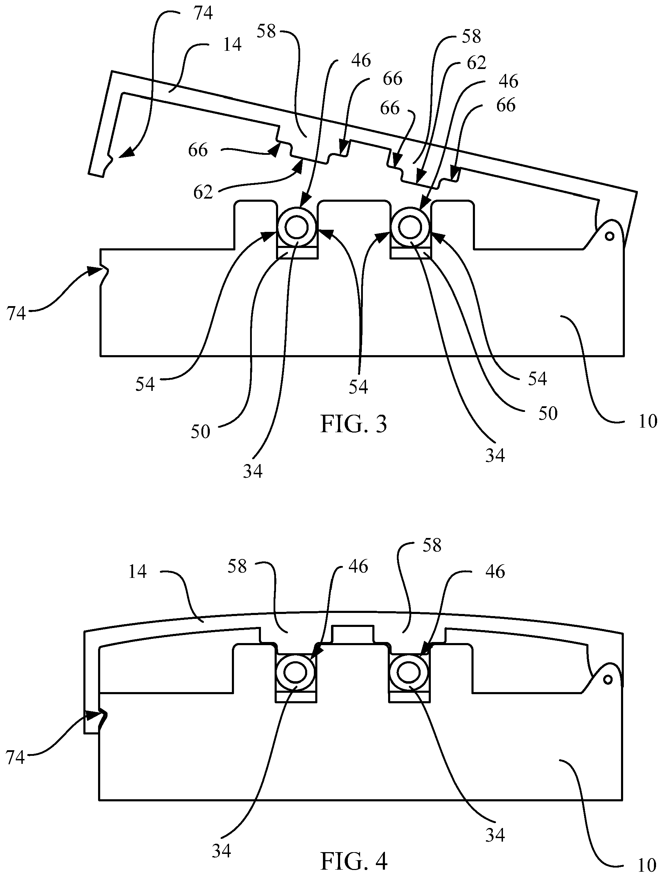

FIG. 3 shows a partial cross-sectional view of the pump 10 taken through the pressure monitoring channels 46. For clarity, not all structures are shown. The pump tubing 34 is loaded into the pressure monitoring channels 46. The pump door 14 is shown open. Pressure sensors 50 are located in the bottom of the channels 46. Piezoelectric crystals are typically used for the sensors 50, but other types of pressure sensors could be used. Variances in the pressure within the pump tubing 34 change the amount of force applied to the pressure sensors, providing a signal which may be used to calculate the pressure inside of the tubing 34. The sidewalls 54 of the pressure monitoring channels 46 may contact the tubing 34 in order to constrain the tubing. In this case the sidewalls 54 would be slightly narrower than the outer diameter of the tubing to limit the movement or expansion of the tubing and to slightly compress the tubing. Alternatively, the sidewalls 54 may be spaced apart from the tubing slightly to allow the tubing to more freely press against the pressure sensors 50.

The pump door 14 has pedestals 58 formed thereon which are formed in alignment with the pressure monitoring channels 46. The pedestals 58 extend downwardly from the inside of the door 14. The bottoms of pedestals 58 have a tubing contacting surface 62 and channel contacting surfaces 66. When the door 14 is closed, the tubing contacting surface 62 contacts the top of the tubing 34 and compresses the tubing slightly, pressing the tubing against the pressure sensor 50. When the door 14 is closed, the channel contacting surfaces 66 contact the top of the channels 46 and rest against the channel, preventing the pedestals 58 from moving towards the tubing 34 and further compressing the tubing. The door 14 is pivotably attached to the pump 10 via a hinge 70 and is secured close with a latch or catch 74.

FIG. 4 shows the pump door 14 in the closed position. When the pump door 14 is closed, the projections 58 are pushed down against the tubing 34 and the pressure monitoring channels 46. The projections 58 are made slightly taller than the available distance between the closed pump door 14 and the channels 46, causing interference when closing the pump door. Thus, the projections 58 contact the pressure monitoring channels 46 before the pump door 14 is completely closed and the pump door is bent as shown in order to close the latch 74 and secure the pump door in a closed position. The bend in the door 14 is exaggerated to illustrate the bending of the door. In use, a slight interference and a slight bend in the door 14 is sufficient to ensure that the projections 58 are always disposed in contact with the channels 46. The portion of the pump door 14 adjacent the projections 58 is bowed outwardly relative to the rest of the pump door. This bending of the door biases the projections 58 against the pressure monitoring channels 46 and maintains contact and pressure therebetween. The contact and applied pressure between the channel contacting surfaces 66 of the projections 58 and the pressure monitoring channels 46 prevents the projections 58 from moving relative to the channels 46 when the pump is in use, moved, or grasped by a user, preventing erroneous changes in the pressure reading. Thus, the tubing 34 is held in a consistent position and is consistently held against the pressure sensor 50 with a small amount of preload. This allows for more reliable pressure monitoring.

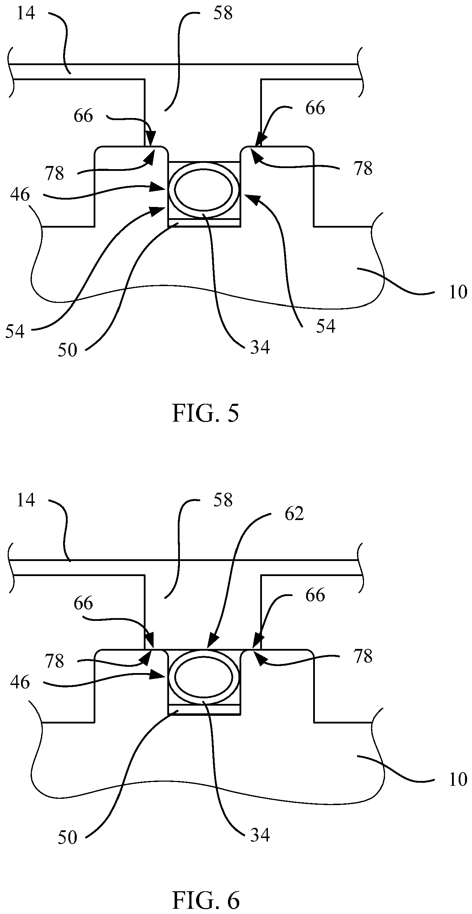

FIG. 5 shows an enlarged view of a single projection 58 and pressure monitoring channel 46 with the pump door 14 in the closed position. The channel contacting surfaces 66 are biased towards and pressed against upper surfaces 78 of the pressure monitoring channel 46. Thus, the contact between the channel contacting surfaces 66 and upper channel surfaces 78 prevents the projection 58 from moving further towards the tubing 34 and further compressing the tubing if a person grabs the pump 10. The tubing contacting surface 62 presses against the tubing 34 and compresses the tubing slightly. In this configuration, the tubing 34 is contacted on four sides by the projection 58, channel side walls 54, and pressure sensor 50. As discussed above, the channel side walls 54 may be slightly wider than the tubing such that the tubing contacts the projection 58 and pressure sensor 50. Because the tubing 34 is loaded consistently, more accurate and consistent pressure readings are obtained. If the tubing 34 is constrained on all sides, expansive force due to pressure within the tube may be more fully directed towards the pressure sensor 50. If the tubing 34 is not contacted by the side walls 54, the tubing may more easily seat against the pressure sensor 50 and eliminate friction with the side walls as a source of error.

FIG. 6 shows an alternate configuration where the tubing contacting surface 62 and the channel contacting surfaces 66 are at or near the same height, or in the same plane. In this configuration, the pressure monitoring channel 46 is made slightly shallower so that the tubing 34 protrudes slightly from the channel 46 before the pump door 14 is closed, causing the tubing contacting surface 62 to press the tubing 34 downwardly when the door 14 is closed. As discussed above, the door 14 is slightly bent when fully closed to bias the projection 58 towards the channel 46 and maintain pressure between the channel contacting surfaces 66 and upper surfaces of the channel 46.

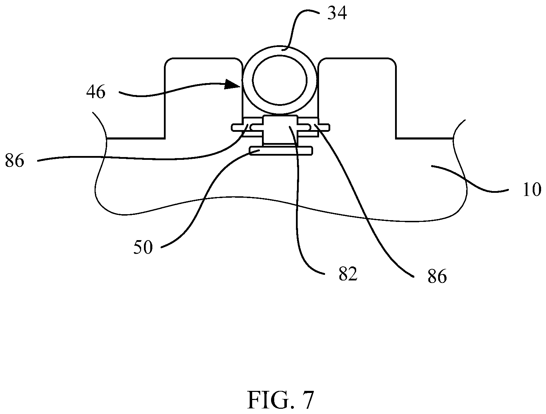

FIG. 7 shows an alternate configuration where the pressure sensor 50 is separated from the tubing 34. A rigid intermediate connecting member 82 is placed therebetween to transfer force between the tubing 34 and the pressure sensor 50. The connecting member 82 is coupled to the pump 10 by a flexible membrane 86, allowing the connecting member to move relative to the pump body and transfer force from the tubing to the pressure sensor 50. The membrane 86 seals around the connecting member 82 and isolates the pressure sensor 50 from the exterior of the pump, making the pump easier to clean and less likely to become damaged due to liquid spills around the pump. The pressure sensor configuration of FIG. 7 functions with the projection 58 as discussed above.

The pressure sensor configuration shown is advantageous in allowing for more consistent pressure measurements. The tube 34 is held against the pressure sensor 50 with a consistent amount of preload by the projection 58. The projection 58 is held against the channel with a consistent amount of preload by the slightly bent door 14, but is prevented from moving further towards the channel 46 and tube 34 by the channel contacting surfaces 66. In this manner, the tube 34 is held in a consistent position where it is unaffected by external influences such as movement of the pump or pressure placed on the pump door. Thus, the pressure sensing is more accurate where the pump is used in an ambulatory (carried with the person) application, where the pump is moved about with a hospital bed, or where a person must move the pump around.

It will be appreciated that various aspects of the disclosure may be combined together. In accordance with the present invention, a pressure monitoring system for a pump includes: a pump having a pressure monitoring channel; a tubing disposed in the pressure monitoring channel; a pressure sensor disposed in communication with the tubing to monitor the pressure in the tubing; a pump door; and a projection disposed on the inside of the pump door, the projection engaging the tubing and the pressure monitoring channel when the pump door is closed, and wherein closing the door causes a portion of the door adjacent the projection to bend outwardly and thereby bias the projection towards the pressure monitoring channel. The pressure monitoring system may also include the projection having a channel contacting surface which contacts the channel when the door is closed to thereby prevent further movement of the projection towards the channel; the channel contacting surface contacting an upper surface adjacent the channel; and/or the projection having a tubing contacting surface on the bottom thereof, the tubing contacting surface contacting the tubing and compressing the tubing when the door is closed; or combinations thereof.

In accordance with one aspect of the disclosure, a pressure monitoring system may include: a pump having a channel therein for receiving a flexible tubing; a tubing disposed in the channel; a pressure sensor disposed in communication with the tubing; a pump door; a projection on the pump door; and wherein, when the pump door is closed: the projection is moved adjacent the channel; the projection compresses the tubing into the channel; the projection contacts a pump surface to stop movement of the projection towards the tubing; and the projection is biased towards the tubing. The pressure monitoring system may further include a portion of the door adjacent the projection being bent outwardly when the door is closed to thereby bias the projection towards the tubing; the projection having a tubing contacting surface for contacting the surface and a channel contacting surface which contacts the channel to thereby stop movement of the projection towards the tubing; the projection having first and second channel contacting surfaces, and the first channel contacting surface contacting a first side of the channel and the second channel contacting surface contacting a second side of the channel opposite the first side; and/or channel contacting surface contacting a surface adjacent the top of the channel; or combinations thereof.

In accordance with an aspect of the disclosure, a pressure monitoring system may include a channel; a flexible tube disposed in the channel, the flexible tube being expandable due to pressure; a pressure sensor disposed in communication with the tube; a projection disposed in contact with the channel and in contact with the tube to hold the tube in the channel. The pressure monitoring system may also include: the projection having a channel contacting surface which contacts the channel to prevent movement of the projection towards the channel; the projection having a tube contacting surface which holds the tube in the channel; the tube contacting surface pressing the tube against the pressure sensor; the tube contacting surface extending into the channel; the channel being part of a pump; the projection being formed as part of a pump door; the projection having an interference fit between the pump door and the channel, causing the pump door to bend when the pump door is closed; the projection being biased towards the channel; and/or a channel contacting surface and preventing movement of the projection towards the channel; or combinations thereof.

There is thus disclosed an improved pressure monitoring system. It will be appreciated that numerous changes may be made to the present invention without departing from the scope of the claims.

## Claims

1. A pressure monitoring system comprising:
a channel (46);
a flexible tube (34) disposed in the channel, the flexible tube being expandable due to pressure;
a pressure sensor (50) disposed in communication with the tube;
a projection (58) disposed in contact with the channel (46) and in contact with the tube (34) to hold the tube (34) in the channel (46), wherein the projection (58) has a channel contacting surface (66) which contacts the channel to prevent movement of the projection (58) towards the channel (46); and
a pump (10) comprising the channel (46), wherein the channel (46) is a pressure monitoring channel and wherein the pressure sensor (50) is disposed in communication with the tube (34) to monitor the pressure in the tube (34);
**characterised in that**
the pressure monitoring system further comprises a pump door (14);
the projection (58) is formed as part of the pump door (14);
the projection (58) is disposed on the inside of the pump door (14), the projection (58) engaging the tube (34) and the pressure monitoring channel when the pump door (14) is closed,
the projection (58) is an interference fit between the pump door (14) and the channel (46), causing the pump door (14) to bend when the pump door (14) is closed, wherein closing the door causes a portion of the door adjacent the projection (58) to bend outwardly and thereby bias the projection (58) towards the pressure monitoring channel.

2. The system of claim 1, wherein the projection (58) has a tube contacting surface (62) which holds the tube (34) in the channel (46).

3. The system of claim 2, wherein the tube contacting surface (62) presses the tube (34) against the pressure sensor (50).

4. The system of claim 2 or 3, wherein the tube contacting surface (62) extends into the channel (46).

5. The system of any preceding claim, wherein the projection (58) is biased towards the channel (46).

6. The system of claim 1, wherein the projection (58) has a channel contacting surface (66) and wherein the channel contacting surface (66) contacts the channel (46) when the door (14) is closed to thereby prevent further movement of the projection (58) towards the channel (46).

7. The system of claim 6, wherein the channel contacting surface (62) contacts an upper surface (78) adjacent the channel (46).

8. The system of claim 7 or 8, wherein the projection (58) has a tube contacting surface (62) on the bottom thereof, the tube contacting surface (62) contacting the tube and compressing the tube (34) when the door (14) is closed.

9. The system of claim 1
wherein, when the pump door (14) is closed:
the projection (58) is moved adjacent the channel (46);
the projection (58) compresses the tube (34) into the channel (46);
the projection (58) contacts a pump surface to stop movement of the projection (58) towards the tube (34); and
the projection (58) is biased towards the tube (34).

10. The system of claim 9, wherein a portion of the door adjacent the projection (58) is bent outwardly when the door (14) is closed to thereby bias the projection (58) towards the tube (34).

11. The system of claim 9 or 10, wherein the projection (58) comprises a tube contacting surface (62) for contacting the surface and a channel contacting surface (66) which contacts the channel to thereby stop movement of the projection (58) towards the tube (34).

12. The system of claim 11, wherein the projection (58) comprises first and second channel contacting surfaces, and wherein the first channel contacting surface contacts a first side of the channel and the second channel contacting surface contacts a second side of the channel opposite the first side.

13. The system of claim 11 or 12, wherein the channel contacting surface (66) contacts a surface adjacent the top of the channel (46).

## Patentansprüche

1. Drucküberwachungssystem, umfassend:
einen Kanal (46);
ein flexibles Rohr (34), das in dem Kanal angeordnet ist, wobei das flexible Rohr unter Druck expandierbar ist;
einen Drucksensor (50), der in Verbindung mit dem Rohr angeordnet ist;
einen Vorsprung (58), der in Kontakt mit dem Kanal (46) und in Kontakt mit dem Rohr (34) angeordnet ist, um das Rohr (34) in dem Kanal (46) zu halten, wobei der Vorsprung (58) eine Kanalkontaktfläche (66) aufweist, die den Kanal berührt, um die Bewegung des Vorsprungs (58) in Richtung des Kanals (46) zu verhindern; und
eine Pumpe (10), die den Kanal (46) umfasst, wobei der Kanal (46) ein Drucküberwachungskanal ist und wobei der Drucksensor (50) in Verbindung mit dem Rohr (34) angeordnet ist, um den Druck in dem Rohr (34) zu überwachen;
**dadurch gekennzeichnet, dass**
das Drucküberwachungssystem ferner eine Pumpentür (14) umfasst;
der Vorsprung (58) als Teil der Pumpentür (14) ausgebildet ist;
der Vorsprung (58) auf der Innenseite der Pumpentür (14) angeordnet ist, wobei der Vorsprung (58) in das Rohr (34) und den Drucküberwachungskanal eingreift, wenn die Pumpentür (14) geschlossen ist,
der Vorsprung (58) eine Presspassung zwischen der Pumpentür (14) und dem Kanal (46) ist, die bewirkt, dass sich die Pumpentür (14) verbiegt, wenn die Pumpentür (14) geschlossen ist, wobei das Schließen der Tür bewirkt, dass sich ein Abschnitt der Tür angrenzend an den Vorsprung (58) nach außen biegt und dadurch den Vorsprung (58) in Richtung des Drucküberwachungskanals vorspannt.

2. System nach Anspruch 1, wobei der Vorsprung (58) eine Rohrkontaktfläche (62) aufweist, die das Rohr (34) in dem Kanal (46) hält.

3. System nach Anspruch 2, wobei die Rohrkontaktfläche (62) das Rohr (34) gegen den Drucksensor (50) drückt.

4. System nach Anspruch 2 oder 3, wobei sich die Rohrkontaktfläche (62) in den Kanal (46) erstreckt.

5. System nach einem der vorstehenden Ansprüche, wobei der Vorsprung (58) in Richtung des Kanals (46) vorgespannt ist.

6. System nach Anspruch 1, wobei der Vorsprung (58) eine Kanalkontaktfläche (66) aufweist und wobei die Kanalkontaktfläche (66) den Kanal (46) berührt, wenn die Tür (14) geschlossen ist, um dadurch eine weitere Bewegung des Vorsprungs (58) in Richtung des Kanals (46) zu verhindern.

7. System nach Anspruch 6, wobei die Kanalkontaktfläche (62) eine obere Oberfläche (78) angrenzend an den Kanal (46) berührt.

8. System nach Anspruch 7 oder 8, wobei der Vorsprung (58) eine Rohrkontaktfläche (62) auf deren Unterseite aufweist, wobei die Rohrkontaktfläche (62) das Rohr berührt und das Rohr (34) zusammendrückt, wenn die Tür (14) geschlossen ist.

9. System nach Anspruch 1, wobei, wenn die Pumpentür (14) geschlossen wird:
der Vorsprung (58) angrenzend an den Kanal (46) bewegt wird;
der Vorsprung (58) das Rohr (34) in den Kanal (46) drückt;
der Vorsprung (58) eine Pumpenfläche berührt, um die Bewegung des Vorsprungs (58) in Richtung des Rohres (34) anzuhalten; und
der Vorsprung (58) in Richtung des Rohres (34) vorgespannt ist.

10. System nach Anspruch 9, wobei ein Abschnitt der Tür angrenzend an den Vorsprung (58) nach außen gebogen wird, wenn die Tür (14) geschlossen wird, um dadurch den Vorsprung (58) in Richtung des Rohres (34) vorzuspannen.

11. System nach Anspruch 9 oder 10, wobei der Vorsprung (58) eine Rohrkontaktfläche (62) zum Berühren der Oberfläche und eine Kanalkontaktfläche (66) umfasst, die den Kanal berührt, um dadurch die Bewegung des Vorsprungs (58) in Richtung des Rohres (34) anzuhalten.

12. System nach Anspruch 11, wobei der Vorsprung (58) erste und zweite Kanalkontaktflächen umfasst, und wobei die erste Kanalkontaktfläche eine erste Seite des Kanals berührt und die zweite Kanalkontaktfläche eine zweite Seite des Kanals gegenüber der ersten Seite berührt.

13. System nach Anspruch 11 oder 12, wobei die Kanalkontaktfläche (66) eine angrenzend an die Oberseite des Kanals (46) berührt.

## Revendications

1. Système de surveillance de la pression comprenant :
un canal (46) ;
un tube flexible (34) disposé dans le canal, le tube flexible étant expansible en raison de la pression ;
un capteur de pression (50) disposé en communication avec le tube ;
une saillie (58) disposée en contact avec le canal (46) et en contact avec le tube (34) pour maintenir le tube (34) dans le canal (46), dans lequel la saillie (58) a une surface de contact de canal (66) qui entre en contact avec le canal pour empêcher le mouvement de la saillie (58) vers le canal (46) ; et
une pompe (10) comprenant le canal (46), dans lequel le canal (46) est un canal de surveillance de la pression et dans lequel le capteur de pression (50) est disposé en communication avec le tube (34) pour surveiller la pression dans le tube (34) ;
**caractérisé en ce que**
le système de surveillance de la pression comprend en outre une porte de pompe (14) ;
la saillie (58) est formée comme partie intégrante de la porte de pompe (14) ;
la saillie (58) est disposée à l'intérieur de la porte de pompe (14), la saillie (58) venant en prise avec le tube (34) et le canal de surveillance de la pression lorsque la porte de pompe (14) est fermée,
la saillie (58) est un ajustement serré entre la porte de pompe (14) et le canal (46), amenant la porte de pompe (14) à se courber lorsque la porte de pompe (14) est fermée, dans lequel la fermeture de la porte amène une partie de la porte à côté de la saillie (58) à se courber vers l'extérieur et de ce fait à solliciter la saillie (58) vers le canal de surveillance de la pression.

2. Système selon la revendication 1, dans lequel la saillie (58) a une surface de contact de tube (62) qui maintient le tube (34) dans le canal (46).

3. Système selon la revendication 2, dans lequel la surface de contact de tube (62) presse le tube (34) contre le capteur de pression (50).

4. Système selon la revendication 2 ou 3, dans lequel la surface de contact de tube (62) s'étend dans le canal (46).

5. Système selon une quelconque revendication précédente, dans lequel la saillie (58) est sollicitée vers le canal (46).

6. Système selon la revendication 1, dans lequel la saillie (58) a une surface de contact de canal (66) et dans lequel la surface de contact de canal (66) est en contact avec le canal (46) lorsque la porte (14) est fermée pour de ce fait empêcher un mouvement supplémentaire de la saillie (58) vers le canal (46).

7. Système selon la revendication 6, dans lequel la surface de contact de canal (62) entre en contact avec une surface supérieure (78) à côté du canal (46).

8. Système selon la revendication 7 ou 8, dans lequel la saillie (58) a une surface de contact de tube (62) sur la partie inférieure de celui-ci, la surface de contact de tube (62) entrant en contact avec le tube et comprimant le tube (34) lorsque la porte (14) est fermée.

9. Système selon la revendication 1, dans lequel, lorsque la porte de pompe (14) est fermée :
la saillie (58) est déplacée à côté du canal (46) ;
la saillie (58) comprime le tube (34) dans le canal (46) ;
la saillie (58) entre en contact avec une surface de pompe pour arrêter le mouvement de la saillie (58) vers le tube (34) ; et
la saillie (58) est sollicitée vers le tube (34).

10. Système selon la revendication 9, dans lequel une partie de la porte à côté de la saillie (58) est courbée vers l'extérieur lorsque la porte (14) est fermée pour de ce fait solliciter la saillie (58) vers le tube (34).

11. Système selon la revendication 9 ou 10, dans lequel la saillie (58) comprend une surface de contact de tube (62) pour entrer en contact avec la surface et une surface de contact de canal (66) qui entre en contact avec le canal pour de ce fait arrêter le mouvement de la saillie (58) vers le tube (34).

12. Système selon la revendication 11, dans lequel la saillie (58) comprend des première et seconde surfaces de contact de canal, et dans lequel la première surface de contact de canal entre en contact avec un premier côté du canal et la seconde surface de contact de canal entre en contact avec un second côté du canal opposé au premier côté.

13. Système selon la revendication 11 ou 12, dans lequel la surface de contact de canal (66) entre en contact avec une surface à côté de la partie supérieure du canal (46).
